# EUROPÄISCHE PATENTSCHRIFT

(11) **EP 3 825 423 B1**
(45) Veröffentlichungstag und Bekanntmachung des Hinweises auf die Patenterteilung: **07.06.2023**
(21) Anmeldenummer: 20208349.9
(22) Anmeldetag: 18.11.2020
(51) Int. Cl.: C14B 1/26, C14B 17/00, D06H 3/04

(54) **VORRICHTUNG ZUM STRECKEN VON TIERHAUT**
DEVICE FOR STRETCHING HIDES
DISPOSITIF D'ÉTENDAGE POUR DES PEAUX D'ANIMAUX

(30) Priorität: 25.11.2019 AT 510242019
(43) Veröffentlichungstag der Anmeldung: 26.05.2021
(73) Patentinhaber: Wollsdorf International GmbH, 8181 Wollsdorf (AT)
(72) Erfinder: Hitrec, Dean, 49 250 Zlatar (HR); Laskowska, Katarzyna, 8200 Gleisdorf (AT)
(74) Vertreter: Röggla, Harald

(56) Entgegenhaltungen:
- EP-A1- 2 105 529
- DE-A1- 10 133 020
- DE-B3-102006 002 999

## Beschreibung

Die Erfindung betrifft eine Vorrichtung zum Strecken von einem, oder zum gleichzeitigen Strecken von mehreren Einzelzuschnitten aus Tierhaut gemäß dem Oberbegriff des Anspruchs 1.

Bei der Lederherstellung wird in einer Abfolge von mechanischen und chemischen Prozessen aus einer Tierhaut eine fertige Lederhaut erzeugt. Da Leder in einer Vielzahl unterschiedlicher Anwendungen eingesetzt wird, muss vor bzw. nach verschiedenen Prozessschritten während der Lederherstellung die Qualität der Tierhaut bestimmt werden, um festzulegen, für welche Anwendung die fertige Lederhaut oder Teilbereiche der Lederhaut geeignet sind. Der finanzielle Wert einer Lederhaut ist ebenfalls wesentlich durch die Qualität der fertigen Lederhaut geprägt.

Die Inspektion der Lederhäute erfolgt nach dem Stand der Technik mittels visuellen Inspektionsmethoden, wobei die Inspektion entweder manuell von einem Menschen, oder automatisiert, mittels eines Kamerasystems durchgeführt wird. Bei der Qualitätskontrolle von Tierhaut kommen mehrere unterschiedliche Prüfeinrichtungen zur Prüfung unterschiedlicher Qualitätskriterien zum Einsatz. Mit einem so genannten Taber bzw. Veslic oder Martindale Prüfgerät wird die Abriebfestigkeit geprüft. Mit einem so genannten Flexometer wird die Knickbeständigkeit geprüft. Bei einem Flammtest wird die Enflammbarkeit geprüft. Zu Leder veredelte Tierhaut wird Licht und UV-Strahlung ausgesetzt, um die Lichtechtheit zu prüfen. Mit einem weiteren Gerät wird eine Farbmessung der Farbe der Oberfläche der Tierhaut durchgeführt. Des Weiteren wird die zu Leder fertig verarbeitete Tierhaut auf Festigkeit (z.B. Zugfestigkeit, Weiterreisskraft, Dehnbarkeit) geprüft. Abschießend findet eine manuelle und visuelle Endkontrolle statt, bei der im Wesentlichen die Qualität bezüglich Hautschädigungen des Leders als Qualitätskriterium beurteilt wird.

Bei beispielsweise Rindsleder sind eine Vielzahl unterschiedlicher Arten von Hautschädigungen bekannt, die auf Verletzungen, Flechten, Milben, Risse, Gabelstöße und andere Ursachen zurückzuführen sind. Jede Art dieser Hautschädigungen hat eine typische Ausbildung, wobei Gabelstöße beispielsweise einen kleinen runden Umfang und Hautschädigungen durch Mastfalten längliche und parallel verlaufende Umrisse aufweisen.

Im Rahmen der Qualitätskontrolle wird, um Schädigungen an Tierhäuten einfacher zu erkennen, die Tierhaut gestreckt, wodurch die oben genannten Schädigungen beziehungsweise Qualitätsmerkmale verstärkt zu Tage treten, und somit einfacher erkannt werden können. Hierdurch erhöht sich die Genauigkeit der Qualitätskontrolle in der Lederherstellung.

Zum Strecken von Tierhäuten ist aus dem Stand der Technik bekannt, die Tierhaut manuell unter einem erheblichen Kraftaufwand, oder unter Zuhilfenahme eines Kniehebelspanners über einen Verformungskörper gezogen wird. Insbesondere bei kleinen Lederteilen, wie sie beispielsweise in der Automobilindustrie für die Herstellung von Lederlenkrädern verwendet werden, ist hierbei zur Erreichung der erforderlichen Dehnungswerte, ein großer Kraftaufwand seitens des Personals der in der Qualitätskontrolle erforderlich. Diese ständige mechanische Belastung führt bei dem Personal vermehrt zu physiologischen Schäden und Abnutzungserscheinungen am Bewegungsapparat. Personen, welche diese Tätigkeit ausführen, leiden nach längerer Beschäftigung in diesem Bereich in Folge dessen häufig an irreversiblen gesundheitlichen Schädigungen, und können diese Tätigkeit nicht länger ausführen. Des Weiteren wird durch die manuelle Ausübung von Kraft keine einheitliche Prüfung gewährleistet.

Aus der EP 2 105 529 A1 ist eine Vorrichtung zur Qualitätskontrolle von Leder oder anderen folienartigen Materialien mit einer Einrichtung zur Klemmung des Materials an zwei beabstandeten Klemmstellen, und mit einer beweglichen Prüfnorm bekannt.

Es ist die Aufgabe der vorliegenden Erfindung eine Vorrichtung zum Strecken von Tierhaut zu bilden, welche die Nachteile des Standes der Technik vermeidet.

Erfindungsgemäß wird die vorliegende Aufgabe mit einer Vorrichtung zum Strecken von einem, oder zum gleichzeitigen Strecken von mehreren Einzelzuschnitten aus Tierhaut mit den Merkmalen von Anspruch 1 gelöst.

Die erfindungsgemäße Vorrichtung zum Strecken von einem, oder zum gleichzeitigen Strecken von mehreren Einzelzuschnitten aus Tierhaut umfasst eine, eine Ausnehmung aufweisende Auflagefläche, und zumindest ein Paar an Klemmmitteln. Die Klemmmittel sind dazu ausgebildet, die Tierhaut beziehungsweise den oder die Einzelzuschnitte aus Tierhaut an der Auflagefläche anliegend, sowie die Ausnehmung überspannend, zu fixieren. Vorzugsweise sind die Klemmmittel dazu ausgebildet, die Tierhaut beziehungsweise die Einzelzuschnitte an die Auflagefläche anzupressen. Hierdurch wird ein Verrutschen des oder der Einzelzuschnitte aus Tierhaut verhindert. Vorzugsweise sind die Klemmmittel zudem an zwei gegenüberliegenden Seiten der Ausnehmung der Auflagefläche angeordnet. Des Weiteren umfasst die Vorrichtung einen, durch die Ausnehmung der Auflagefläche in Richtung der, die Ausnehmung überspannenden Tierhaut beweglichen Verformungskörper. Im Betrieb der erfindungsgemäßen Vorrichtung wird mittels den Klemmmitteln eine Tierhaut, wie beispielsweise ein Einzelzuschnitt aus Leder, an der Auflagefläche, vorzugsweise glatt aufliegend befestigt. Die Tierhaut beziehungsweise der Einzelzuschnitt überspannt im befestigten Zustand die Ausnehmung in der Auflagefläche. Im Anschluss wir der Verformungskörper in Richtung der Tierhaut verlagert, wobei der Verformungskörper die Ausnehmung in der Auflagefläche durchtritt. Hierdurch wird der Vorteil erreicht, dass eine reproduzierbare Verformung der Tierhaut erzeugt wird, wodurch Verbesserungen in der Qualitätskontrolle erreicht werden. Die Qualitätskontrolle wird insbesondere dadurch verbessert, da die erreichbare Inspektionsposition der Einzelzuschnitte der späteren Verbausituation am Endprodukt, wie beispielsweise einem Lenkrad, deutlich mehr entspricht, als in aus dem Stand der Technik bekannten Vorrichtungen. Des Weiteren wird hierdurch der notwendige Kraftaufwand im Rahmen der Qualitätskontrolle für das Bedienpersonal, insbesondere bei der Kontrolle kleinflächiger Lederzuschnitte, reduziert.

Vorzugsweise umfasst die erfindungsgemäße Vorrichtung einen mit der Auflagefläche verbundenen Standrahmen. Hierdurch wird verhindert, dass die Auflagefläche der Vorrichtung im Betrieb verrutscht, und ein sicherer Stand der Vorrichtung gewährleistet.

Gemäß der bevorzugten Ausführungsform der erfindungsgemäßen Vorrichtung umfasst die Vorrichtung einen mit dem Verformungskörper verbundenen Pneumatik- oder Hydraulikzylinder. Hierdurch wird der Vorteil erreicht, dass der Verformungskörper mit einer höheren Kraft als bei manueller Bedienung der erfindungsgemäßen Vorrichtung gegen die Tierhaut gedrückt werden kann, wodurch eine verbesserte Nachweiswahrscheinlichkeit für Schädigungen an der Tierhaut erreicht wird. Des Weiteren entfällt hierdurch die Notwendigkeit einer manuellen Ausübung von Kraft durch den Benutzer der erfindungsgemäßen Vorrichtung. Vorzugsweise ist der Pneumatik- oder Hydraulikzylinder mit dem Standrahmen verbunden.

Erfindungsgemäß ist der Verformungskörper dazu ausgebildet ist, die Ausnehmung in der Auflagefläche im Wesentlichen auszufüllen. Hierdurch wird ein Spalt zwischen dem Verformungskörper und der Auflagefläche minimiert, wodurch das Verletzungsrisiko für den Benutzer der erfindungsgemäßen Vorrichtung reduziert wird.

Vorzugsweise sind die Klemmmittel dazu ausgebildet, eine Mehrzahl an Tierhäuten überlappungsfrei an der Auflagefläche anliegend, sowie die Ausnehmung überspannend, zu fixieren. Hierdurch können mehrere Tierhäute beziehungsweise Lederzuschnitte gleichzeitig mit der erfindungsgemäßen Vorrichtung gestreckt werden.

Gemäß der bevorzugten Ausführungsform der erfindungsgemäßen Vorrichtung umfasst jedes der Klemmmittel einen Hebel und einen mit dem Hebel verbundenen Klemmabschnitt. Hierdurch wird der Vorteil erreicht, dass die Tierhaut beziehungsweise die Lederzuschnitte sicher und mit geringem Kraftaufwand an der erfindungsgemäßen Vorrichtung befestigt werden können.

Vorteilhafte Ausgestaltungen der erfindungsgemäßen Vorrichtung sowie alternative Ausführungsvarianten werden in weiterer Folge anhand der Figuren näher erläutert.
Figur 1 zeigt eine perspektivische Ansicht einer ersten Ausführungsvariante einer erfindungsgemäßen Vorrichtung zum Strecken von Tierhaut.
Figur 2 zeigt die erfindungsgemäße Vorrichtung in einer alternativen Ausführungsvariante in einer Ansicht von oben.

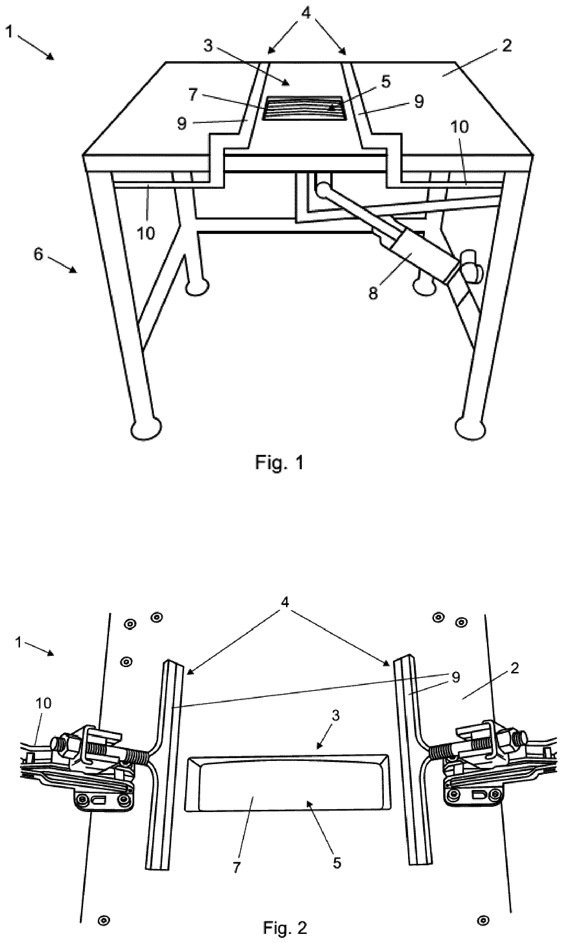

Figur 1 zeigt eine erfindungsgemäße Vorrichtung 1 zum Strecken von einem, oder zum gleichzeitigen Strecken von mehreren Einzelzuschnitten aus Tierhaut in einer ersten Ausführungsvariante. Die erfindungsgemäße Vorrichtung 1 umfasst eine Auflagefläche 2, welche eine Ausnehmung 3 aufweist. Des Weiteren umfasst die erfindungsgemäße Vorrichtung 1 gemäß der bevorzugten Ausführungsform ein Paar an Klemmmitteln 4, wobei auch eine Mehrzahl an Paaren von Klemmmitteln 4 an der erfindungsgemäßen Vorrichtung 1 vorgesehen sein können. Die Klemmmittel 4 fixieren im Betrieb der Vorrichtung 1 eine in den Figuren nicht dargestellte Tierhaut anliegend an der Auflagefläche 2, wobei die Tierhaut im fixierten Zustand die Ausnehmung 3 der Auflagefläche 2 überspannt. Die Vorrichtung 1 weist zudem einen durch die Ausnehmung 3 der Auflagefläche 2 in Richtung der, die Ausnehmung 3 überspannenden Tierhaut beweglichen Verformungskörper 5 auf. Durch die erfindungsgemäße Ausgestaltung der Vorrichtung 1 wird der Vorteil erreicht, dass eine reproduzierbare Verformung der Tierhaut gewährleistet wird, wodurch Verbesserungen in der Qualitätskontrolle erzielt werden. Des Weiteren wird hierdurch der notwendige Kraftaufwand im Rahmen der Qualitätskontrolle für das Bedienpersonal, insbesondere bei der Kontrolle kleinflächiger Lederzuschnitte reduziert. Wie in Figur 1 ersichtlich umfasst die erfindungsgemäße Vorrichtung 1 gemäß der bevorzugten Ausführungsform einen Standrahmen 6, welcher mit der Auflagefläche 2 verbunden ist. Hierdurch ist ein sicherer Stand der Vorrichtung 1 gewährleistet. Die Klemmmittel 4 pressen die Tierhaut vorzugsweise an die Auflagefläche 2 an. Hierdurch wird eine flache und faltenfreie Auflage der Tierhaut auf der Auflagefläche 2 gewährleistet. Der Verformungskörper 5 weist vorzugsweise eine konvex gekrümmte Oberfläche 7 auf, welche im Betrieb der erfindungsgemäßen Vorrichtung 1 durch die Ausnehmung 3 der Auflagefläche 2 hervortritt, und eine Kraft auf die Tierhaut ausübt. Hierdurch wird die Tierhaut entsprechend der Form der Oberfläche 7 verformt. Der Verformungskörper 5 füllt die Ausnehmung 3 in der Auflagefläche 2 vorzugsweise im Wesentlichen aus. Konvexe Oberflächenkrümmungen werden insbesondere bei der Prüfung von Tierhäuten angewendet, welche für die Verwendung an Lederlenkrädern vorgesehen sind. In alternativen Ausführungsformen der erfindungsgemäßen Vorrichtung 1 kann der Verformungskörper 5 auch andere Oberflächenkrümmungen aufweisen, welche vorzugsweise im Wesentlichen zumindest abschnittsweise einer Form eines Objekts entsprechen, welches mit der zur prüfenden Tierhaut überzogen werden soll.

Gemäß der bevorzugten Ausführungsform der erfindungsgemäßen Vorrichtung 1 umfasst diese einen, mit dem Verformungskörper 5 verbundenen, Pneumatik- oder Hydraulikzylinder 8. Hierdurch wird eine präzise Steuerung der auf die Tierhaut aufgebrachten Kraft ermöglicht. Des Weiteren ermöglicht eine Bestimmung einer Längenänderung des Pneumatik- oder Hydraulikzylinders 8 bei einem gegebenen Arbeitsdruck beziehungsweise bei einer gegebenen ausgeübten Kraft Rückschlüsse auf die Dehnungseigenschaften der mit der erfindungsgemäßen Vorrichtung 1 geprüften Tierhaut. Vorzugsweise ist der Pneumatik- oder Hydraulikzylinder 8, wie in Figur 1 dargestellt, mit dem Standrahmen 6 verbunden. Gemäß einer alternativen Ausführungsvariante kann anstatt des Pneumatik- oder Hydraulikzylinders 8 auch ein elektrischer Zahnstangenantrieb vorgesehen sein. In Umrechnung zu einem Hub des Pneumatik- oder Hydraulikzylinders 8 können mit der erfindungsgemäßen Vorrichtung Dehnungswerte der Tierhaut von bis zu 20% erreicht werden.

Figur 2 zeigt die erfindungsgemäße Vorrichtung 1 in einer alternativen Ausführungsvariante, mit alternativen Klemmmitteln 4. Die Klemmmittel 4 weisen vorzugsweise Klemmabschnitt 9 und einen mit dem Klemmabschnitt 9 verbundenen Hebel 10 auf. In der in Figur 2 dargestellten Ausführungsvariante sind die Klemmabschnitte 9 mit einem weichen Filz- oder Schaumelement versehen, welches im Betrieb der erfindungsgemäßen Vorrichtung 1 eine von den Klemmelementen 4 ausgeübte Klemmkraft auf die Tierhaut zu übertragen. Hierdurch wird die Klemmkraft auf eine größere Fläche der Tierhaut verteilt, wodurch Beschädigungen an der Tierhaut vorgebeugt wird. Durch die erfindungsgemäße Ausgestaltung der Klemmmittel 4 mit einem Hebel 10 und einem mit dem Hebel 10 verbundenen Klemmabschnitt 9 wird gewährleistet, dass die Klemmmittel 4 eine hohe Klemmkraft ohne Beschädigungsrisko für den zu prüfenden Lederzuschnitt bereitstellen, und dennoch für einen Benutzer der erfindungsgemäßen Vorrichtung 1 einfach und mit einem geringen Kraftaufwand bedienbar sind.

Wie aus den Figuren ersichtlich, sind die Klemmmittel 4 vorzugsweise an zwei gegenüberliegenden Seiten der Ausnehmung 3 der Auflagefläche 2 angeordnet. Hierdurch wird eine gleichmäßige Spannung der Tierhäute gewährleistet. Zudem fixieren die Klemmmittel 4 gemäß einer in Figur 1 dargestellten bevorzugten Ausführungsvariante eine Mehrzahl an Tierhäuten überlappungsfrei an der Auflagefläche 2 anliegend, sowie die Ausnehmung 3 überspannend. Hierdurch können mehrere Tierhäute gleichzeitig mit der erfindungsgemäßen Vorrichtung 1 geprüft werden, wodurch der Prüfdurchsatz erhöht wird. Vorzugsweise sind bis zu vier Einzelzuschnitte aus einer Tierhaut pro Prüfzyklus mit der erfindungsgemäßen Vorrichtung 1 überprüfbar.

Wird die erfindungsgemäße Vorrichtung 1 entsprechend dimensioniert, ist diese auch für jegliche Lederbaugruppen, die Einzelzuschnitte aus Leder verwenden, anwendbar.

## Patentansprüche

1. Vorrichtung (1) zum Strecken von einem, oder zum gleichzeitigen Strecken von mehreren Einzelzuschnitten aus Tierhaut, wobei die Vorrichtung (1) zumindest ein Paar an Klemmmitteln (4) und einen Verformungskörper (5) umfasst, **dadurch gekennzeichnet, dass** die Vorrichtung (1) eine, eine Ausnehmung (3) aufweisende Auflagefläche (2) umfasst, wobei die Klemmmittel (4) dazu ausgebildet sind, die Tierhaut an der Auflagefläche (2) anliegend, sowie die Ausnehmung (3) überspannend, zu fixieren, und der Verformungskörper (5) durch die Ausnehmung (3) der Auflagefläche (2) in Richtung der, die Ausnehmung (3) überspannende Tierhaut beweglich ist.

2. Vorrichtung (1) gemäß Anspruch 1, **dadurch gekennzeichnet, dass** die Vorrichtung (1) einen mit der Auflagefläche (2) verbundenen Standrahmen (6) umfasst.

3. Vorrichtung (1) gemäß einem der Ansprüche 1 oder 2, **dadurch gekennzeichnet, dass** die Vorrichtung (1) einen mit dem Verformungskörper (5) verbundenen Pneumatik- oder Hydraulikzylinder (8) umfasst.

4. Vorrichtung (1) gemäß den Ansprüchen 2 und 3, **dadurch gekennzeichnet, dass** der Pneumatik- oder Hydraulikzylinder (8) mit dem Standrahmen (6) verbunden ist.

5. Vorrichtung (1) gemäß einem der Ansprüche 1 bis 4, **dadurch gekennzeichnet, dass** der Verformungskörper (5) dazu ausgebildet ist, die Ausnehmung (3) in der Auflagefläche (2) im Wesentlichen auszufüllen.

6. Vorrichtung (1) gemäß einem der Ansprüche 1 bis 5, **dadurch gekennzeichnet, dass** die Klemmmittel (4) dazu ausgebildet sind, die Tierhaut an die Auflagefläche (2) anzupressen.

7. Vorrichtung (1) gemäß einem der Ansprüche 1 bis 6, **dadurch gekennzeichnet, dass** die Klemmmittel (4) dazu ausgebildet sind, eine Mehrzahl an Tierhäuten überlappungsfrei an der Auflagefläche (2) anliegend, sowie die Ausnehmung (3) überspannend, beschädigungsfrei zu fixieren.

8. Vorrichtung (1) gemäß einem der Ansprüche 1 bis 7, **dadurch gekennzeichnet, dass** die Klemmmittel (4) an zwei gegenüberliegenden Seiten der Ausnehmung (3) der Auflagefläche (2) angeordnet sind.

9. Vorrichtung (1) gemäß einem der Ansprüche 1 bis 8, **dadurch gekennzeichnet, dass** jedes der Klemmmittel (4) einen Hebel (10) und einen mit dem Hebel (10) verbundenen Klemmabschnitt (9) umfasst.

## Claims

1. A device (1) for stretching one or for the simultaneous stretching of several individual blanks of animal skin, wherein the device (1) comprises at least one pair of clamping means (4) and a deformation body (5), **characterized in that** the device (1) comprises a supporting surface (2) having a recess (3), the clamping means (4) being designed for fixing the animal skin such that it lies against the supporting surface (2) and spans the recess (3), and the deformation body (5) is movable through the recess (3) of the supporting surface (2) in the direction of the animal skin spanning the recess (3).

2. A device (1) according to claim 1, **characterized in that** the device (1) comprises a stand frame (6) connected to the supporting surface (2).

3. A device (1) according to any of claims 1 or 2, **characterized in that** the device (1) comprises a pneumatic or hydraulic cylinder (8) connected to the deformation body (5).

4. A device (1) according to claims 2 and 3, **characterized in that** the pneumatic or hydraulic cylinder (8) is connected to the stand frame (6).

5. A device (1) according to any of claims 1 to 4, **characterized in that** the deformation body (5) is designed for essentially filling the recess (3) in the supporting surface (2).

6. A device (1) according to any of claims 1 to 5, **characterized in that** the clamping means (4) are designed for pressing the animal skin against the supporting surface (2).

7. A device (1) according to any of claims 1 to 6, **characterized in that** the clamping means (4) are designed for fixing a plurality of animal skins without damage such that they lie against the supporting surface (2) without overlapping and span the recess (3).

8. A device (1) according to any of claims 1 to 7, **characterized in that** the clamping means (4) are arranged on two opposite sides of the recess (3) of the supporting surface (2).

9. A device (1) according to any of claims 1 to 8, **characterized in that** each one of the clamping means (4) comprises a lever (10) and a clamping section (9) connected to the lever (10).

## Revendications

1. Dispositif (1) pour l'étendage d'un, ou pour l'étendage simultané de plusieurs coupons de peau d'animal, lequel dispositif (1) comprend au moins une paire de moyens de serrage (4) et un corps de déformation (5), **caractérisé en ce que** ce dispositif (1) comprend une surface de support (2) présentant un réceptacle (3), les moyens de serrage (4) étant conçus pour fixer la peau d'animal posée sur la surface de support (2) et enjambant le réceptacle (3), et le corps de déformation (5) pouvant se déplacer à travers la cavité (3) de la surface de support (2) en direction de la peau d'animal enjambant le réceptacle (3).

2. Dispositif (1) selon la revendication 1, **caractérisé en ce que** ce dispositif (1) comprend un châssis (6) relié à la surface de support (2).

3. Dispositif (1) selon une des revendications 1 ou 2, **caractérisé en ce que** ce dispositif (1) comprend un vérin pneumatique ou hydraulique (8) relié au corps de déformation (5).

4. Dispositif (1) selon les revendications 2 et 3, **caractérisé en ce que** le vérin pneumatique ou hydraulique (8) est relié au châssis (6).

5. Dispositif (1) selon une des revendications 1 à 4, **caractérisé en ce que** le corps de déformation (5) est conçu pour remplir sensiblement le réceptacle (3) dans la surface de support (2).

6. Dispositif (1) selon une des revendications 1 à 5, **caractérisé en ce que** les moyens de serrage (4) sont conçus pour presser la peau d'animal sur la surface de support (2).

7. Dispositif (1) selon une des revendications 1 à 6, **caractérisé en ce que** les moyens de serrage (4) sont conçus pour fixer sans les endommager et sans qu'elles se chevauchent une pluralité de peaux d'animaux posées sur la surface de support (2) et enjambant le réceptacle (3).

8. Dispositif (1) selon une des revendications 1 à 7, **caractérisé en ce que** les moyens de serrage (4) sont disposés sur deux côtés opposés du réceptacle (3) de la surface de support (2).

9. Dispositif (1) selon une des revendications 1 à 8, **caractérisé en ce que** chacun des moyens de serrage (4) comprend un levier (10) et une section de serrage (9) reliée au levier (10).
